(19) Europäisches Patentamt
European Patent Office
Office européen des brevets

(11) EP 1 195 373 B1

(12) **EUROPEAN PATENT SPECIFICATION**

(45) Date of publication and mention
of the grant of the patent:
**23.03.2005 Bulletin 2005/12**

(51) Int Cl.[7]: **C07D 233/88**, G01N 30/02

(21) Application number: **01123251.9**

(22) Date of filing: **02.10.2001**

(54) **Method for separation of 5-hydroxycreatinine**

Verfahren zur Trennung von 5-Hydroxycreatinin

Méthode pour la séparation de 5-hydroxycréatinine

(84) Designated Contracting States:
**AT BE CH CY DE DK ES FI FR GB GR IE IT LI LU MC NL PT SE TR**

(30) Priority: **03.10.2000 JP 2000303172**

(43) Date of publication of application:
**10.04.2002 Bulletin 2002/15**

(73) Proprietor: **NIPPON ZOKI PHARMACEUTICAL CO., LTD.**
**Chuo-ku, Osaka (JP)**

(72) Inventors:
• **Nakamura, Ko, Institute of Bio-Active Science**
**Yashiro-cho, Katoh-gun, Hyogo (JP)**
• **Kawano, Katsumi**
**Hachioji-shi, Tokyo (JP)**

(74) Representative: **HOFFMANN - EITLE**
**Patent- und Rechtsanwälte**
**Arabellastrasse 4**
**81925 München (DE)**

(56) References cited:
• **DATABASE CA [Online] CHEMICAL ABSTRACTS SERVICE, COLUMBUS, OHIO, US; HIGASHIDATE, SAKAE ET AL: "Rapid and highly sensitive method for the determination of guanidino compounds in body fluids" retrieved from STN Database accession no. 101:106722 XP002190360 & BUNSEKI KAGAKU (1984), 33(7), 366-70 ,**
• **DATABASE WPI Section Ch, Week 199409 Derwent Publications Ltd., London, GB; Class A13, AN 1994-071150 XP002190361 & JP 06 023279 A (BABCOCK-HITACHI KK), 1 February 1994 (1994-02-01)**

## Description

### Field of the Invention

The present invention relates to a method for the separation of 5-hydroxycreatinine (5-HC), which is useful in the determination of this compound in a test to detect a variety of diseases, especially renal function disorders and systemic oxidative stress.

### Prior Art

5-HC is known as an intermediate in the production of methylguanidine, which is one of the main urinary toxins accumulated in blood of patients suffering from renal failure. It has been shown that 5-HC is produced by non-enzymatic oxidation of creatinine. 5-HC is not detected in serum of healthy persons, but it is detected from serum of persons suffering from renal failure from its initial stage on, and its level increases with the severity of the symptoms. Thus, 5-HC has occupied the attention as a marker for renal function disorder and its determination has been considered valuable to evaluate the disease condition and to detect renal failure in a patient at an early stage.

It is also believed that hydroxy radicals, which are known as being very reactive, participate in the non-enzymatic oxidation of creatinine. Thus, 5-HC also gets much attention as an index for the formation of hydroxy radicals *in vivo* (index for oxidative stress).

Consequently, it has been suggested that the determination of 5-HC can be useful as a marker for renal function disorder such as renal failure, diabetic nephropathy and nephritis, and also as a marker for systemic oxidative stress.

Regarding the determination of 5-HC as a marker for renal disorders, a method is known described by Nakamura (one of the present inventors) in the Japanese Patent Application Publication Nos. 04/161854 and 05/119038. According to this method 5-HC is separated by HPLC and the resulting 5-HC is hydrolyzed and converted to methylguanidine followed by quantitative determination by means of fluorescent labeling.

However, 5-HC is separated using a column of cation-exchange resin and applying five kinds of separation solvents according to the method described by Higashidate et al. Bunseki Kagaku, 33, 366- 370 (1984). According to this method, the following first to fifth separation solvents are used:

1) sodium citrate/hydrochloric acid (pH 3.00) for 4.5 min.,
2) sodium citrate/hydrochloric acid (pH 3.50) for 2.8 min.,
3) sodium citrate/hydrochloric acid (pH 5.25) for 2.4 min.,
4) sodium citrate/boric acid/sodium hydroxide (pH 10.00) for 2.3 min., and
5) 1M sodium hydroxide for 30 min.

Thus, the above method is disadvantageous due to the need to prepare five different separation solvents in advance, and the method itself is complex and cumbersome, since e.g. programming for changing the separation solvents and controlling the eluting times is required. Moreover, the sensitivity of this method for 5-HC is 0.5-1 nmol/mL (6.5-13 μg/dL), which is not sufficient to detect small changes of 5-HC in a sample derived from a living organism.

Furthermore, Nakamura et al., Nephron, 66, 140-146 (1994) discloses a method of separating and determining 5-HC using a separation solvent comprising 1 part of dimethyl sulfoxide (DMSO) and 9 parts of 0.4M citric acid, adjusted to pH 5.25.

In this method the eluting time is as long as 30 minutes and that is not an efficient method. Also, the sensitivity for the measurement, which is about 2 μg/dL, is still insufficient to measure the small changes of 5-HC concentrations in the initial stage of renal function disorders and is therefore not satisfactory as well.

### Summary of the Invention

In view of the above situation, the present inventors have carried out intensive investigation to find a method to separate 5-HC which overcomes the problems of the prior art.

Thus, an object of the present invention is to provide a highly sensitive and practical method for the separation of 5-HC useful in a test detecting 5-HC as an indicator of renal function disorders, systemic oxidative stress, etc..

As a result of the present inventors' studies, the above problem was solved by providing a method for the separation of 5-HC, comprising the step of subjecting a sample to high performance liquid chromatography (HPLC) separation using a strongly acidic cation-exchange resin of a sulfonic acid type of a styrene-divinylbenzene series and a separation solvent having a pH of 4.1-4.6.

**Brief Description of the Figures**

Fig. 1 is an example of a specific system applicable to perform the method of the present invention.

Fig. 2 shows the constitution of the specific system applicable to perform the method of the present invention as shown in Fig. 1.

Fig. 3 is a graph showing the pH-dependency of the elution time for 5-HC and other contaminants in urine of a healthy person determined in Example 1.

**Detailed Description**

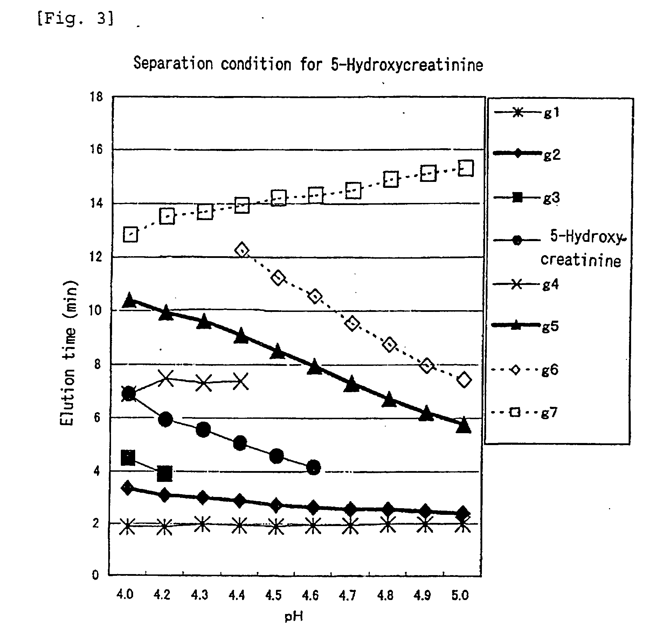

The present invention relates to a method for separation of 5-HC, comprising the step of subjecting a sample to high performance liquid chromatography (HPLC) separation using a strongly acidic cation-exchange resin and a separation solvent having a pH of 4.1-4.6.
Preferably, to detect the 5-HC separated from a sample by the present process, the separated 5-HC is hydrolyzed and converted into methylguanidine, which is then quantitatively determined after previous appropriate fluorescence labeling.
The present invention will be illustrated in detail in the following.
The strongly acidic cation-exchange resin used in the present invention is a cation-exchange resin of a sulfonic acid modified styrene-divinylbenzene resin, i.e. a styrene-divinylbenzene copolymer having sulfonic acid groups introduced into the polymer chains. Such resins are known in the art, and various kinds of it are commercially available, such as "Guanidino Pack II" (trade name, supplied by Nippon Bunko K. K.), which is an example of a preferred strongly acidic cation-exchange resin within the context of the present invention.
The separation solvent used for the HPLC in the present method invention is a solvent having a pH of 4.1-4.6, preferably 4.2-4.3. Examples of preferred solvents are citrate buffer, phosphate buffer, etc. Citrate buffer is more preferred, and can be prepared using sodium citrate, potassium citrate, etc. Its concentration may be appropriately set up and, when sodium citrate is used, a solution concentration of 0.30-0.45M in terms of sodium ions is preferred.
To achieve a high separating ability it is preferred to use a citrate/ dimethyl sulfoxide (DMSO) type mixed solvent as separation solvent, preferably a sodium citrate/DMSO solvent, wherein DMSO is appropriately added to a citrate buffer.
Due to the addition of DMSO, preferred effects such as increase in fluorescence intensity and good separation of elution peaks are obtained. The amount of DMSO is not specifically limited, however, a separation solvent comprising up to 20 wt.-% DMSO is preferred. More preferably, the amount of DMSO is 5-15 wt.-%, most preferably about 10wt.-%.
The pH of the separation solvent is within the range of 4.1-4.6 and is adjusted by addition of an acid. The acid used to this end is not specifically limited, and any acid can be used, which does not influence the final result of the 5-HC determination. As an example hydrochloric acid is preferred. In the determination of 5-HC in a sample derived from animals including man, such as human urine and serum, it is preferred that the pH is selected such that 5-HC is most distinctly separated from other contaminants. As is apparent from the experimental result discussed later (see Example 1 and Fig. 3), a pH of 4.2-4.3 is preferred to achieve separation from big peaks of contaminants eluted before and after 5-HC.
The sample subject to the present method preferably is a body fluid, such as blood, serum or plasma; urine, etc. derived from animals, more preferably from mammals, including man. Most preferably, the sample is derived from man, and is suited as item for clinical tests.

**Examples**

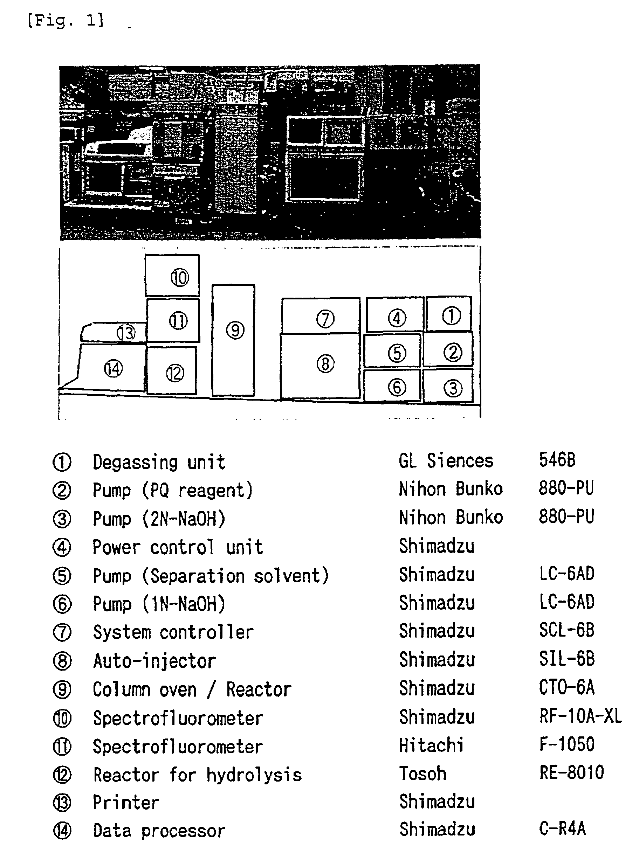

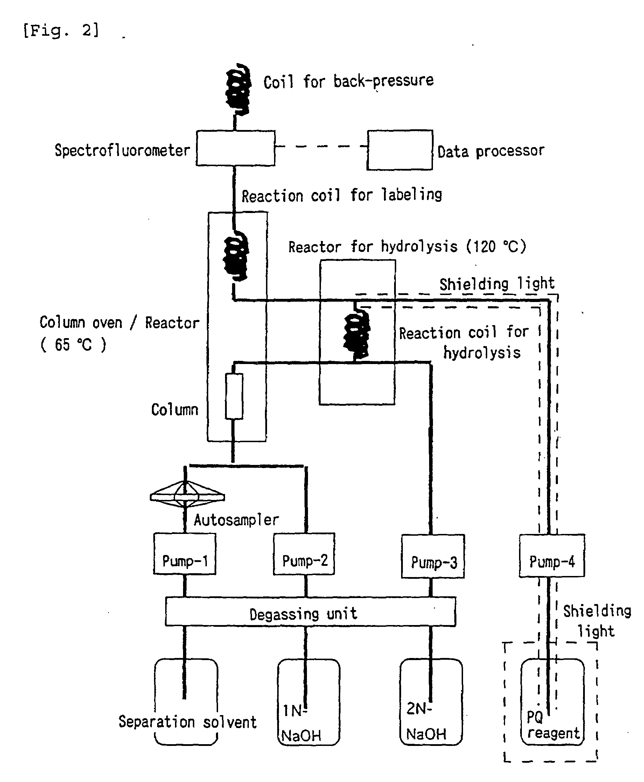

The present invention will now be illustrated more specifically by way of Examples, although the present invention is not limited by them at all.
In the experiments, the separation and detection system shown in Figs. 1 and 2 was used. "Guanidino Pack II" (manufactured by Nippon Bunko K. K.), which is a column having a size of 6.0 mm (ø) × 35.0 mm, was used as strongly acidic cation-exchange resin.

Example 1

Separating conditions of 5-HC.

In a strongly acidic cation-exchange resin, eluting time for the aimed substance varies mostly depending upon the ion concentration and the pH. Thus, to evaluate the eluting time dependency on the pH, mixed solvents comprising 9 parts of 0.4M sodium citrate solution and 1 part of DMSO was added with different amounts of hydrochloric acid to vary the pH thereof within the range of 3-5. These solvents were different only with regard to the amount of hydrochloric acid added.

As a sample deproteinated urine from a healthy person was used. The above solvents having different pH values were used as separating solvents. Switching to 1N sodium hydroxide was not carried out but only separation solvent was used. By using the solvents having different ph values in individual experiments, the resulting change in the eluting pattern for 5-HC and other constituents of the sample was checked.

The results obtained for a pH range of 4-5 are shown in Fig. 3. Symbols g1-g7 were assigned to the peaks of contaminants other than that of 5-HC. Bold lines in the drawing mean big peaks. As can be seen, for some contaminants the elution time decreases with increasing pH, while others show an increase of the elution time with increasing pH. Also, in some cases almost no pH dependency of the elution time was observed.

The result of the above experiments shows that the peaks of 5-HC and of other contaminants, respectively, were most efficiently separated at a pH of about 3.6 and 4.3. Since at a pH of 4.3 the eluting time for 5-HC is shorter, which is preferred for practical reasons, a separation solvent adjusted to pH 4.3 was used in the following experiments.

Example 2

Calibration Curve Stability and detection limit of 5-HC

A 5-HC sample was diluted with 10% trichloroacetic acid (TCA) yielding samples having a 5-HC concentration of 20, 10, 4, 2, l, 0,4 and 0,2 µM, respectively. Injection amounts of 0.1 ml of each sample were measured. Each diluted sample was repeatedly measured and the stability of the calibration curve was evaluated. As control, a sample containing no 5-HC was measured, which did not give a detectable peak in any experiment (peak height = 0). A concentration yielding a coefficient of variation (CV) of less than 20% was considered to represent a practically measurable range. The results are shown in Table 1.

As can be drawn from Table 1, according to the present method the data obtained at an infusion rate of 20 pM/0.1 ml (0.2 µM), determination yielded a CV of the read values of far less than 20%, and it is likely that stable data are available until far lower concentrations. This was further verified in Example 3.

[Table 1]

| Run | Concentration of 5-HC (µM) | | | | | | |
|---|---|---|---|---|---|---|---|
| | 20 | 10 | 4 | 2 | 1 | 0,4 | 0,2 |
| | Peak Height (a.U.) | | | | | | |
| 1 | 418.697 | 211.172 | 85.131 | 42.890 | 21.109 | 8.462 | 4.479 |
| 2 | 421.971 | 217.147 | 86.989 | 43.408 | 21.400 | 8.875 | 4.557 |
| 3 | 445.784 | 221.821 | 88.146 | 44.617 | 21.807 | 8.716 | 4.621 |
| 4 | 443.129 | 217.290 | 88.665 | 44.591 | 22.250 | 8.824 | 4.540 |
| 5 | 441.884 | 216.756 | 90.541 | 44.652 | 22.089 | 9.279 | 4.250 |
| 6 | 438.391 | 223.424 | 88.563 | 43.400 | 21.709 | 8.638 | 4.448 |
| 7 | 438.945 | 225.801 | 91.510 | 43.480 | 225.61 | 9.202 | 4.732 |
| mean | 435.543,0 | 219.058,7 | 88.506,4 | 43.862,6 | 21.846,4 | 8.856,6 | 4.516,9 |
| SD | 10726,2 | 4940,1 | 2121,9 | 734,4 | 499,3 | 295,1 | 148,8 |
| CV | 2,46 | 2,26 | 2,40 | 1,67 | 2,29 | 3,33 | 3,29 |
| SD = standard deviation | | | | | | | |
| CV = coefficient of variation | | | | | | | |
| a.U. = arbitrary unit | | | | | | | |

Example 3

Calibration curve stability and detection limit of 5-HC at low concentrations

A 5-HC sample was diluted with 10% TCA yielding samples having a 5-HC concentration of 2, 0.8, 0.4, 0.2, 0.08, 0.04 and 0.02 μM, respectively. Injection amounts of 0.1 ml of each sample were measured. Each diluted sample was repeatedly measured and the stability of the calibration curve was evaluated. As control, a sample containing no 5-HC was measured, which did not give a detectable peak in any experiment (peak height = 0). A concentration yielding a coefficient of variation (CV) of less than 20% was considered to represent a practically measurable range. The results are shown in Table 2.

As shown in Table 2, the present method achieves a CV of the read values of less than 20% even at a concentration as low as 0.02 μM⁻. The calibration curve by means of a least-squares method showed a good linearity as follows.

$$y = (31.339\ x) - (147,63\ R^2\ ) = 0,9988$$

The y intercept is -147.63, corresponding to about 1/5 of the read value for 0.02 μM, which is within the allowable error range from 1SD.

[Table 2]

| Run | Concentration of 5-HC (μM) | | | | | | |
|---|---|---|---|---|---|---|---|
| | 2 | 0.8 | 0.4 | 0.2 | 0.08 | 0.04 | 0.02 |
| | Peak Height (a.U.) | | | | | | |
| 1 | 64.393 | 25.202 | 11.968 | 5.784 | 2.284 | 1.009 | 605 |
| 2 | 59.977 | 24.426 | 12.047 | 5.582 | 2.325 | 1.049 | 640 |
| 3 | 63.323 | 24.931 | 12.542 | 5.663 | 1.990 | 1.185 | 772 |
| 4 | 62.254 | 26.640 | 11.767 | 6.173 | 2.374 | 1.113 | 961 |
| 5 | 63.071 | 23.647 | 11.717 | 6.437 | 2.708 | 1.434 | 796 |
| mean | 62.603.6 | 24.969.2 | 12.008.2 | 5.927.8 | 2.336.2 | 1.158.0 | 754.8 |
| SD | 1.655.2 | 1.105.7 | 328.3 | 363.9 | 256.1 | 168.1 | 141.5 |
| CV | 2.64 | 4.43 | 2.73 | 6.14 | 10.96 | 14.52 | 18.75 |
| SD = standard deviation | | | | | | | |
| CV = coefficient of variation | | | | | | | |

Example 4

Investigation of sensitivity in measurement

The above Examples 2 and 3 show that the measurable range of the present method 0.02-20 μM (infusion of 2-2000 pM/0.1 ml). Also, the calibration curve showed a good linearity from the maximum (20 μM) to the minimum (0.02 μM) concentration, and even to zero concentration. Thus, even if a calibration curve by means of a dilution series is not prepared, a calibration can be performed by a one-point absolute determination at one single concentration, e.g. at 20 μM.

Further, if one takes the 5-HC level in serum of a healthy person into consideration, it can be assumed to be likely that, by reaching a practically useful determination sensitivity of 0.02 μM, nearly all samples which may be subject to investigation in a test are within a measurable range. With regard to samples of more than 20 μM an accurate determination is possible at least after dilution, thus reducing the concentration into the above range.

Effect of the Invention

According to the present method, only one separation solvent is required, and the time necessary to perform the measurement of one analysis cycle is about 14 minutes. This is considerably shorter than in the conventional methods. Thus, with one HPLC-setup about 100 determinations can be carried out per day and, which is about twice as much as compared with the method described by Nakamura et al. discussed above.

Also, as is apparent from the above results, the present method is sufficiently sensitive to allow the accurate determination of 5-HC in a concentration of 0.02 μM (0.26 μg/dl). Thus, even in blood of a healthy person 5-HC can be determined, which shows that the present method is highly sensitive as compared with the methods known in the art.

The reason for the high sensitivity is assumed to be that the time necessary for the analysis is shortened and that, since the separation solvent is more acidic, the decomposition rate of 5-HC is reduced. By combination of these two effects, 5-HC is hardly decomposed during the analysis.

Hence, the present method is a very efficient and useful method to separate and determine 5-HC in a sample, which allows for practical applications which have been impossible with the conventional methods.

## Claims

1. Method for the separation of 5-hydroxycreatinine, comprising the step of subjecting a sample to high performance liquid chromatography (HPLC) separation using a strongly acidic cation-exchange resin of a sulfonic acid type of a styrene-divinylbenzene series and a separation solvent having a pH of 4.1-4.6.

2. Method of claim 1, wherein the separation solvent is selected from citrate buffer solvents, phosphate buffer solvents and mixed citrate buffer/dimethyl sulfoxide solvents.

3. Method of any of the preceding claims, wherein the solvent has a pH within the range of 4.2-4.3.

4. Method of any of the preceding claims, wherein hydrochloric acid is present to adjust the pH to the desired value.

5. Method of any of the preceding claims, wherein the sample subjected to separation is derived from a living body.

6. Method of claim 5, wherein the sample subjected to separation is derived from blood, serum or urine.

7. Method of claim 5 or 6, wherein the living body is a mammal, including man.

8. Method of any of the preceding claims, comprising the further steps of hydrolyzing the separated 5-HC and converting it into methylguanidine, and quantitatively determining the methylguanidine after subjecting it to fluorescence labeling.

9. Use of a solvent having a pH of 4.1-4.6, in combination with a strongly acidic cation-exchange resin of a sulfonic acid type of a styrene-divinylbenzene series, for the separation of 5-hydroxycreatinine from a sample by means of high performance liquid chromatography (HPLC).

## Patentansprüche

1. Verfahren zur Abtrennung von 5-Hydroxykreatinin, das den Schritt der Durchführung einer High Performance Liquid Chromatography (HPLC)-Auftrennung mit einer Probe unter Verwendung eines stark sauren Kationenaustauscherharzes vom Sulfonsäuretyp der Styroldivinylbenzolserie und eines Auftrennlösungsmittels mit einem pH-Wert von 4,1-4,6 umfasst.

2. Verfahren gemäss Anspruch 1, worin das Auftrennlösungsmittel ausgewählt ist aus Citratpufferlösungsmitteln, Phosphatpufferlösungsmitteln und gemischten Citratpuffer/Dimethylsulfoxid-Lösungsmitteln.

3. Verfahren gemäss mindestens einem der vorhergehenden Ansprüche, worin das Lösungsmittel einen pH-Wert im Bereich von 4,2-4,3 aufweist.

4. Verfahren gemäss mindestens einem der vorhergehenden Ansprüche, worin zur Einstellung des pH-Werts auf den gewünschten Wert Salzsäure vorhanden ist.

5. Verfahren gemäss mindestens einem der vorhergehenden Ansprüche, worin die der Auftrennung unterworfene Probe aus einem lebenden Körper erhalten wurde.

6. Verfahren gemäss Anspruch 5, worin die der Auftrennung unterworfene Probe aus Blut, Serum oder Urin erhalten wurde.

7. Verfahren gemäss Anspruch 5 oder 6, worin der lebende Körper ein Säuger ist, einschliesslich eines Menschen.

8. Verfahren gemäss mindestens einem der vorhergehenden Ansprüche, das ferner die Schritte der Hydrolyse des abgetrennten 5-HC und dessen Umwandlung in Methylguanidin und die quantitative Bestimmung des Methylguanidins, nachdem dieses fluoreszenzmarkiert wurde, umfasst.

9. Verwendung eines Lösungsmittels mit einem pH-Wert von 4,1-4,6 in Kombination mit einem stark sauren Kationenaustauscherharz vom Sulfonsäuretyp der Styroldivinylbenzolserie zur Abtrennung von 5-Hydroxykreatinin aus einer Probe mittels High Performance Liquid Chromatography (HPLC).

**Revendications**

1. Procédé pour la séparation de 5-hydroxycréatinine, comprenant l'étape de soumettre un échantillon à une séparation par chromatographie liquide à haute performance (HPLC) en utilisant une résine échangeuse de cation fortement acide d'un type acide sulfonique d'une série styrène-divinylbenzène et un solvant de séparation ayant un pH de 4,1-4,6.

2. Procédé de la revendication 1, dans lequel le solvant de séparation est choisi parmi des solvants tampons au citrate, des solvants tampons au phosphate, et des solvants mixtes tampons au citrate/diméthylsulfoxyde.

3. Procédé de l'une quelconque des revendications précédentes, dans lequel le solvant a un pH dans la gamme de 4,2-4,3.

4. Procédé de l'une quelconque des revendications précédentes, dans lequel de l'acide chlorhydrique est présent afin d'ajuster le pH à la valeur désirée.

5. Procédé de l'une quelconque des revendications précédentes, dans lequel l'échantillon soumis à la séparation provient d'un corps vivant.

6. Procédé de la revendication 5, dans lequel l'échantillon soumis à la séparation est dérivé du sang, du sérum ou de l'urine.

7. Procédé de la revendication 5 ou 6, dans lequel le corps vivant est un mammifère, incluant un homme.

8. Procédé de l'une quelconque des revendications précédentes, comprenant les autres étapes d'hydrolyser la 5-HC séparée et de la convertir en méthylguanidine, et de déterminer quantitativement la méthylguanidine après l'avoir soumise à un marquage par fluorescence.

9. Utilisation d'un solvant ayant un pH de 4,1-4,6, en combinaison avec une résine échangeuse de cations fortement acide d'un type acide sulfonique d'une série styrène-divinylbenzène, pour la séparation de 5-hydroxycréatinine à partir d'un échantillon au moyen d'une chromatographie liquide à haute performance (HPLC).

[Fig. 1]

| | | | |
|---|---|---|---|
| ① | Degassing unit | GL Siences | 546B |
| ② | Pump (PQ reagent) | Nihon Bunko | 880-PU |
| ③ | Pump (2N-NaOH) | Nihon Bunko | 880-PU |
| ④ | Power control unit | Shimadzu | |
| ⑤ | Pump (Separation solvent) | Shimadzu | LC-6AD |
| ⑥ | Pump (1N-NaOH) | Shimadzu | LC-6AD |
| ⑦ | System controller | Shimadzu | SCL-6B |
| ⑧ | Auto-injector | Shimadzu | SIL-6B |
| ⑨ | Column oven / Reactor | Shimadzu | CTO-6A |
| ⑩ | Spectrofluorometer | Shimadzu | RF-10A-XL |
| ⑪ | Spectrofluorometer | Hitachi | F-1050 |
| ⑫ | Reactor for hydrolysis | Tosoh | RE-8010 |
| ⑬ | Printer | Shimadzu | |
| ⑭ | Data processor | Shimadzu | C-R4A |

[Fig. 2]

Coil for back-pressure

Spectrofluorometer — Data processor

Reaction coil for labeling

Reactor for hydrolysis (120 °C)

Shielding light

Column oven / Reactor ( 65 °C )

Reaction coil for hydrolysis

Column

Autosampler

Pump-1    Pump-2    Pump-3    Pump-4

Degassing unit

Shielding light

Separation solvent    1N-NaOH    2N-NaOH    PQ reagent

[Fig. 3]

Separation condition for 5-Hydroxycreatinine